# FASCICULE DE BREVET EUROPEEN

(11) **EP 0 375 582 B1**
(45) Date de publication et mention de la délivrance du brevet: **26.05.1993**
(21) Numéro de dépôt: 89470021.0
(22) Date de dépôt: 14.11.1989
(51) Int. Cl.: A61F 2/38

(54) **Dispositif d'ancrage pour prothèse de genou**
Verankerungsvorrichtung für Knieprothese
Anchoring device for a knee prosthesis

(30) Priorité: 20.12.1988 FR 8817159
(43) Date de publication de la demande: 27.06.1990
(73) Titulaire: S.P.O.R.T., F-54670 Custines (FR)
(72) Inventeur: Mansat, Christian, F-31130 Balma (FR)
(74) Mandataire: Poupon, Michel

(56) Documents cités:
- EP-A- 0 295 200
- DE-A- 2 631 351
- FR-A- 2 171 321
- FR-A- 2 585 236
- FR-A- 2 593 390
- GB-A- 719 308
- US-A- 3 869 731
- US-A- 3 916 451
- US-A- 4 285 070

## Description

La présente invention a pour objet un perfectionnement apporté à une prothèse de genou de type monocompartimentale ou bicompartimentale, comportant un dispositif d'ancrage et un plateau tibial muni sur sa face d'appui tibiale de rainures d'ancrage à section en queue d'aronde formant quadrillage.

Des prothèses de ce type sont en elles-mêmes connues.

Elles ont par exemple fait l'objet du brevet FR-A-2 585 236 auquel il est fait ici expressément référence en tant qu'état de la technique pour les systèmes d'ancrage existants.

Les rainures du plateau tibial sont destinées à augmenter l'ancrage à l'aide d'un ciment sur le tibia, après formation d'un plateau sur celui-ci.

On constate que l'ancrage obtenu n'est pas toujours satisfaisant.

Conformément à l'invention, on remédie à cet inconvénient en proposant un perfectionnement apporté à une prothèse de genou de type monocompartimentale ou bicompartimentale comportant un dispositif d'ancrage et un plateau tibial muni sur sa face d'appui tibiale de rainures d'ancrage à section en queue d'aronde formant quadrillage, caractérisé en ce que l'ancrage est réalisé par un croisillon d'ancrage inséré dans le tibia et muni à sa face supérieure d'un tenon complémentaire de la rainure formant mortaise et venant s'insérer dans celle-ci.

De préférence, les ailes du croisillon seront munies d'orifices permettant un régénérescence osseuse au travers dudit croisillon.

Cette structure permet à la fois une assise du plateau tibial et un verrouillage de celui-ci.

On comprendra mieux l'invention à l'aide de la description faite ci-après d'un mode non limitatif de mise en oeuvre en référence aux dessins annexés dans lesquels :

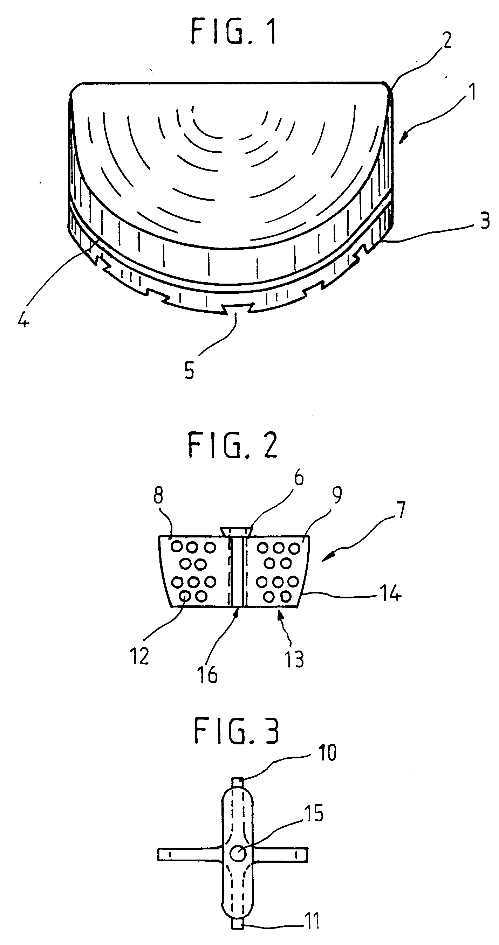

- la figure 1 est une vue en perspective d'un plateau tibial ;
- la figure 2 est une élévation latérale d'un croisillon conforme à l'invention ;
- la figure 3 est une vue de dessus d'un croisillon conforme à l'invention.

Le plateau tibial, généralement référencé (1) peut être soit en matière synthétique monobloc, soit composé d'une plaque supérieure (2) synthétique et d'une plaque inférieure métallique (3).

Dans le cas d'un plateau monobloc synthétique une gorge (4) reçoit un fil métallique pour la localisation du plateau par radiographie.

Sur sa face venant en appui sur le tibia, le plateau comporte des rainures (5) en queue d'aronde qui forment un quadrillage, à angles droits ou non.

Conformément à l'invention, ces rainures coopèrent avec un tenon (6) ménagé sur un croisillon (7) comportant une pluralité, par exemple quatre, d'ailes planes (8,9,10,11) disposées en croix.

Les parois de ces ailes sont pourvues d'orifices (12) qui permettent le passage de l'os régénéré.

Ce croisillon est inséré à force dans la face supérieure nettoyée et aplanie du tibia devant recevoir le plateau tibial.

Afin de faciliter son insertion, le croisillon pourra posséder des arêtes (13) légèrement biseautées, les chants latéraux (14) allant se rétrécissant vers le bas. Cette insertion sera également facilitée par un alésage central (15) débouchant sur l'autre face en (16).

Ce croisillon pourra être réalisé en tout matériau, par exemple en titane.

On peut également envisager, de manière tout à fait exceptionnelle, d'utiliser ce croisillon sans solidarisation avec le plateau tibial.

Il présente plusieurs avantages tels que :
- ancrage et fixation du plateau tibial par verrouillage naturel osseux ;
- assise de plateau tibial par réhabilitation osseuse ;
- régénérescence du plateau tibial naturel par sollicitation.osseuse.

## Revendications

1. Prothèse de genou de type monocompartimentale ou bicompartimentale comportant un dispositif d'ancrage et un plateau tibial (1) muni sur sa face d'appui tibiale de rainures d'ancrage (5) à section en queue d'aronde formant quadrillage, caractérisé en ce que l'ancrage est réalisé par un croisillon d'ancrage (7) insérable dans le tibia et muni à sa face supérieure d'un tenon (6) complémentaire de la rainure formant mortaise et venant s'insérer dans celle-ci.

2. Prothèse de genou selon la revendication 1, caractérisé en ce que le croisillon (7) comporte une pluralité d'ailes planes (8,9,10,11).

3. Prothèse de genou selon la revendication 2, caractérisé en ce que les parois des ailes (8,9,10,11) sont pourvues d'orifices (12).

4. Prothèse de genou selon l'une quelconque des revendications 1 à 3, caractérisé en ce que le croisillon possède des arêtes (13) légèrement biseautées, les chants latéraux (14) allant se rétrécissant vers le bas et un alésage central (15) débouchant sur l'autre face en (16).

## Claims

1. Knee prosthesis of the mono-compartmental or bi-compartmental type, comprising a securing device and a tibial plate (1), which is provided, on its tibial supporting face, with securing grooves (5) of dovetail cross-section forming a square configuration, characterised in that the securement is achieved by a securing cross-piece member (7), which is insertable into the tibia and is provided, on its upper face, with a tenon (6) complementing the groove forming a mortise and being inserted therein.

2. Knee prosthesis according to claim 1, characterised in that the cross-piece member (7) comprises a plurality of planar vanes (8, 9, 10, 11).

3. Knee prosthesis according to claim 2, characterised in that the walls of the vanes (8, 9, 10, 11) are provided with apertures (12).

4. Knee prosthesis according to any of claims 1 to 3, characterised in that the cross-piece member has slightly bevelled edges (13), the lateral sides (14) narrowing downwardly and a central bore (15) extending to the other face at (16).

## Patentansprüche

1. Knieprothese der Einkammer- oder Zweikammerbauart, welche eine Verankerungsvorrichtung und eine schienbeinseitige Scheibe (1) aufweist, die auf ihrer das Schienbein abstützenden Seite im Querschnitt schwalbenschwanzförmige, ein Raster bildende Nuten (5) für die Verankerung aufweist, **dadurch gekennzeichnet,** daß die Verankerung durch ein in das Schienbein einsetzbares Verankerungskreuz (7) gebildet ist, welches auf seiner oberen Seite einen entsprechend der eine Führung bildenden Nut geformten Mitnehmer (6) hat und welcher in sie einsetzbar ist.

2. Knieprothese nach Anspruch 1, **dadurch gekennzeichnet,** daß das Verankerungskreuz (7) eine Vielzahl von ebenen Stegen (8, 9, 10, 11) aufweist.

3. Knieprothese nach Anspruch 2, **dadurch gekennzeichnet,** daß die Wände der Stege (8, 9, 10, 11) mit Löchern (12) versehen sind.

4. Knieprothese nach einem der Ansprüche 1 bis 3, **dadurch gekennzeichnet,** daß das Kreuz leicht abgeschrägte Anschläge (13) aufweist, deren seitliche Kanten (14) sich nach unten hin nähern und daß sie eine mittige Bohrung (15) aufweist, welche bei (16) aus der anderen Seite herausführt.
